# EUROPÄISCHE PATENTSCHRIFT

(11) **EP 0 343 275 B1**
(45) Veröffentlichungstag und Bekanntmachung des Hinweises auf die Patenterteilung: **03.02.1993**
(21) Anmeldenummer: 88108458.6
(22) Anmeldetag: 27.05.1988
(51) Int. Cl.: A61K 35/16, A61L 2/16, C07K 3/28

(54) **Verfahren zur Herstellung eines hochreinen, virusfreien Antihämophiliefaktors mittels Chromatographie**
Process for the production of a high purity, virus-free antihaemophilic factor by means of chromatography
Procédé de préparation d'un facteur antihémophilique hautement purifié exempt de virus au moyen de chromatographie

(43) Veröffentlichungstag der Anmeldung: 29.11.1989
(73) Patentinhaber: CENTRE REGIONAL DE TRANSFUSION SANGUINE DE LILLE, 59012 Lille (FR)
(72) Erfinder: Smith, Andrew, CH-8750 Glarus (CH); Burnouf, Myriana Centre Regional de, F-59000 Lille (FR)
(74) Vertreter: Lepeudry-Gautherat, Thérèse

(56) Entgegenhaltungen:
- EP-A- 0 173 242
- EP-A- 0 238 701
- EP-A- 0 239 859
- US-A- 4 478 825

## Beschreibung

Die Erfindung betrifft ein Verfahren zur Herstellung eines hochreinen, durch Behandlung mit biokompatiblen organischen Lösungsmitteln/ Detergenzien virusfreien Antihämophiliefaktors (AHF oder Faktor VIII) durch Reinigung eines Kyropräzipitats.

In der EP-A-0 238 701 wird ein Verfahren zur Herstellung eines hochreinen, virusfreien Antihämophiliefaktors beschrieben, wobei das Kryopräzipitat mittels Ethanolfällung von Fibrinogen, Globulinen, Albuminen und anderen störenden Bestandteilen befreit wird. Die Anreicherung aus dem Kryopräzipitat ist notwendig, da Faktor VIII nur in äußerst geringen Mengen in dem Material enthalten ist. Dieser Anreicherungsschritt beeinträchtigt jedoch den Gehalt an AHF im Endprodukt. Die bisher bekannten Verfahren zur Herstellung von Faktor VIII ergeben nur geringe Mengen an wirksamer Substanz. Bei der Applikation des auf herkömmlichem Wege hergestellten Faktors VIII belastet man den Patienten daher mit großen Mengen antigener Substanzen. Diese Verfahrensweise ist nicht unbedenklich. Es hat daher nicht an Versuchen gefehlt, den Faktor VIII durch Trennungsoperationen weiter anzureichern. So wurde versucht, mittels Affinitätschromatographie mit gegen Faktor VIII gerichteten tierischen Antikörpern Produkte mit höherer spezifischer Aktivität zu erhalten. Diese Technik ist jedoch sehr aufwendig und kostenintensiv. Zum anderen ist sie auch deshalb nicht unbedenklich, da stets eine gewisse Menge tierischen Eiweißes bei jeder chromatographischen Trennung aus der Säule gewaschen wird.

Die der Erfindung zugrunde liegende Aufgabe ist also das Bereitstellen eines Verfahrens zur Herstellung eines hochreinen, durch Behandlung mit biokompatiblen organischen Lösungsmitteln/Detergenzien virusfreien Antihämophiliefaktors (AHF oder Faktor VIII) durch Reinigung eines Kryopräzipitats, wobei sowohl die Ausbeute an Faktor VIII hoch bleibt als auch eine höhere biologische Aktivität pro Gewichtseinheit Protein erzielt wird.

In der EP-A-0 173 242 wird ein Verfahren zur Herstellung einer Präparation des Blutgerinnungsfaktors VIII beschrieben, das erlaubt, ein pasteurisiertes und von Immunoglobulinen, Fibronectin und Fibrinogen praktisch freies Präparat zu gewinnen. Dennoch enthält diese Herstellung kein spezifisches Virusinaktivierungsmittel.

Vor der Befreiung des Kryopräzipitats von Viren wird das aufgetaute Kryopräzipitat mit 1 bis 3 U/ml Heparin enthaltendem Wasser bei pH 6,5 bis 7,5 extrahiert und mit einer Aluminiumhydroxid-Suspension versetzt und nach Abkühlung auf 10 bis 18°C und Einstellen des pH-Wertes auf 6 bis 7 zentrifugiert oder filtriert.

Erfindungsgemäß wird eine höhere Ausbeute an Faktor VIII mit höherer spezifischer Aktivität erzielt, indem nach der Entfernung der Viren die Probe einer Gelpermeationschromatographie an Ionenaustauschermaterialien unterworfen wird.

Woods, K. und Orme, Th. beschreiben in der EP-A-0 239 859, daß es von Vorteil ist, nach der Virusentfernung oder -inaktivierung und vor der chromatographischen Trennung die Probe mit Ölen zu extrahieren, vorzugsweise mit Sojaöl, Rizinöl und/oder Baumwollsamenöl.

Durch die erfindungsgemäßen Maßnahmen ist es erstmals möglich, in hohen Ausbeuten einen hochreinen Antihämophiliefaktor herzustellen, der eine bisher nicht erreichte spezifische Aktivität aufweist. Die bisherigen Ergebnisse deuten darauf hin, daß durch die bisher übliche Ethanolfällung ein beträchtlicher Anteil des Faktors VIII irreversibel zerstört wird.

Handelsübliches Kryopräzipitat wird bei Raumtemperatur innerhalb von 3 bis 4 Stunden aufgetaut und in Stücke von ca. 1 bis 2 cm zerteilt. Diese Stücke werden bei Temperaturen zwischen 10 und 25°C durch Rühren suspendiert in etwa dem doppelten Volumen Wasser, welches 1 bis 3 U/ml Heparin-Natrium enthält. Die Suspension wird mit 0,1 M Essigsäure auf einen pH-Wert von mindestens 7,0 bis 8, vorzugsweise 7,0 bis 7,1 eingestellt. Es wird 15 bis 60 Minuten, vorzugsweise 30 Minuten bei Raumtemperatur gerührt. Daraufhin werden etwa 108 g 2% Aluminiumhydroxid-Suspension pro kg Kryopräzipitat hinzugefügt und 1 bis 10 Minuten, vorzugsweise 5 Minuten bei Raumtemperatur gerührt. Mit Säure, vorzugsweise 0,1 M Essigsäure, wird der Säuregehalt auf pH 6,0 bis 7,0, vorzugsweise 6,5 bis 6,6 eingestellt. Die Probe wird auf 10 bis 18°C, vorzugsweise 14 bis 16°C gekühlt. Man zentrifugiert bei dieser Temperatur, beispielsweise in einer Sharples AS-16 (Cepa 61)-Zentrifuge mit einer Rate von 1,0 L/min. Danach schließt sich eine Filtration des Überstandes, beispielsweise durch ein Pall AB-1 U010ZP-Filter an. Vorzugsweise nach dem Abzentrifugieren und Filtrieren erfolgt eine Virusinaktivierung. Besonders bewährt hat sich die Virusinaktivierung mit Hilfe von Tween^{(R)}/TNBP (Tri-n-Butylphosphat). Gute Ergebnisse werden auch mit Natriumcholat/TNBP erzielt. Das Tween^{(R)}/TNBP- bzw. Natriumcholat/TNBP-Gemisch kann beispielsweise durch Ölextraktion wieder entfernt werden.

Man gibt die Probe auf eine Chromatographiesäule, die ein Gelpermeationsmaterial mit Ionenaustauscheraktivität aufweist, wie beispielsweise Fractogel®-DEAE. Fractogel® ist ein hydrophiles Chromatographiematerial auf Basis von Copolymerisaten aus Oligoethylenglycolen, Glycidylmethacrylaten und Pentaerythroldimethacrylaten. Die Säulenkapazität soll vorzugsweise so beschaffen sein, daß 0,5 kg des Säulenmaterials pro kg Kryopräzipitat sich in der Säule befinden. Die Säule wird mit der Probe beschickt und mit Puffern gewaschen. Nach Elution der Probe mit einem Puffer höherer Ionenstärke wird das erhaltene Produkt mit einem Puffer mit niedrigem Salzgehalt verdünnt und, falls nötig, auf pH 6,5 bis 7,5, vorzugsweise 6,9 bis 7,1 eingestellt. Danach wird erneut filtriert, vorzugsweise an Nitrocellulosefiltern, worauf sich eine Sterilfiltration anschließt.

Das Verfahren wird anhand der folgenden Beispiele näher erläutert.

### Beispiel 1

### Anreicherung des Faktor VIII

Handelsübliches Kryopräzipitat wird bei Raumtemperatur innerhalb von 3 bis 4 Stunden aufgetaut und in Stücke von ca. 1 bis 2 cm zerteilt. Diese Stücke werden bei 20 bis 25°C durch Rühren suspendiert und in etwa dem doppelten Volumen Wasser, welches 2 U/ml Heparin-Natrium enthält, suspendiert. Die Suspension wird mit 0,1 M Essigsäure auf einen pH-Wert von 7,0 bis 7,1 eingestellt. Man rührt 30 Minuten bei 20 bis 25°C. 108 g 2% Aluminiumhydroxid-Suspension pro kg Kryopräzipitat werden hinzugefügt und weitere 5 Minuten bei Raumtemperatur gerührt. Danach stellt man den pH-Wert mit 0,1 M Essigsäure auf pH 6,5 bis 6,6 ein. Danach kühlt man die Probe auf 14 bis 16°C ab, zentrifugiert in einer Sharples AS-16 (Cepa 61)-Zentrifuge mit einer Rate von 1,0 L/min. Der Überstand wird durch ein Pall AB-1 U010ZP-Filter filtriert.

### Beispiel 2

### Vorbereitung der Chromatographiesäule

Zur Trennung des extrahierten Kryopräzipitats wird eine Säule mit mindestens 0,5 l Fractogel®-DEAE Harz pro kg Kryopräzipitat verwendet. Die Säulenhöhe soll ≦ Durchmesser sein. Nach dem Beschicken der Säule mit Harz wird die Chromatographiesäule mit 5 Volumina 0,1 M Natriumchloridlösung gewaschen. Danach wäscht man mit einem Puffer A, der wie folgt zusammengesetzt ist:
110 mM Natriumchlorid, 10 mM Natriumcitrat-5H₂O, 120 mM Glycin, 1 mM Calciumchlorid-2H₂O, pH-Wert 6,9 bis 7,0, mit 1 M HCl einzustellen.

Sämtliche Puffer müssen virusfrei sein, da die folgenden Operationen mit virusfreien Extrakten des Kryopräzipitats durchgeführt werden.

### Beispiel 3

Die Probe wird auf die Säule aufgetragen und die Absorption des Durchflusses bei einer Wellenlänge von 280 nm beobachtet. Das Filtrat wird aufgenommen und auf Faktor VIII-Aktivität untersucht, ebenso wie das Produkt vor der Säulentrennung. Danach wird die Säule mit dem Puffer A gewaschen, bis die Absorption wieder ihren Ausgangswert erreicht. Danach wird die Säule mit Puffer B gewaschen. Dies geschieht so lange, bis die Absorption wieder die Grundlinie erreicht hat.

Der Puffer B hat folgende Zusammensetzung:
160 mM Natriumchlorid, 10 mM Natriumcitrat-5H₂O, 120 mM Glycin, 1 mM Calciumchlorid-2H₂O, pH-Wert 6,9 bis 7,0.

Die Elution des Produkts erfolgt mit dem Puffer C. Die nach Zugabe des Puffers C erscheinende Proteinfraktion wird gesammelt. Puffer C weist folgende Zusammensetzung auf:
250 mM Natriumchlorid, 20 mM Natriumcitrat-5H₂O, 80 mM Glycin, 16 mM Lysin, 2,5 mM Calciumchlorid-2H₂O, pH-Wert 6,9 bis 7,0.

Nach Elution der gewünschten Fraktion wird die Säule anschließend mit 5 Volumina des Puffers D, welcher 1 M Natriumchloridlösung enthält, gewaschen.

Die Regenerierung der Säule erfolgt durch Waschen mit 0,1 N NaOH (3 Säulenvolumina), gefolgt durch Waschen der Säule mit 0,1 N Salzsäure (3 Säulenvolumina) und Waschen der Säule mit 5 Säulenvolumina 25% Alkohol in Wasser.

### Beispiel 4

Die gesammelten Fraktionen werden mit Puffer E, bestehend aus
20 mM Natriumcitrat, 80 mM Glycin, 16 mM Lysin,
2,5 mM Calciumchlorid-2H₂O, pH-Wert 6,9 bis 7,1,
so lange verdünnt, bis sie eine Aktivität von 26 oder 35 U/ml aufweisen. Danach wird der pH-Wert, falls nötig, auf 6,9 bis 7,1 eingestellt, worauf sich eine Filtration des Produkts durch 0,45 µm Sealklean-Filter anschließt. Abschließend wird eine weitere Sterilfiltration durchgeführt.

## Patentansprüche

1. Verfahren zur Herstellung eines hochreinen, virusfreien Antihämophiliefaktors (AHF oder Faktor VIII) nach Vorreinigung eines Kryopräzipitats durch Ausziehen mit Heparin enthaltendem Wasser und Aluminiumhydroxid Versetzung und nach Behandlung mit biokompatiblen organischen Lösungsmitteln/Detergenzien, dadurch gekennzeichnet, daß nach der Entfernung der Viren die Probe einer Gelpermeationschromatographie an Ionenaustauschermaterialien unterworfen wird.

## Claims

1. Process for the production of a high-purity virus-free antihaemophilic factor (AHF or factor VIII) after prior purification of a cryoprecipitate by extracting with water containing heparin and adding aluminium hydroxide, and after treatment with biocompatible organic solvents/detergents, characterised in that, after removal of the viruses, the sample is subjected to gel permeation chromatography on ion-exchange materials.

## Revendications

1. Procédé de préparation d'un facteur antihémophilique (AHF ou Facteur VIII) hautement purifié, exempt de virus, après prépurification d'un cryoprécipité par extraction avec de l'eau contenant de l'héparine et addition d'hydroxyde d'aluminium, et après traitement avec des solvants/détergents organiques biocompatibles, caractérisé en ce qu'après l'élimination des virus, l'échantillon est soumis à une chromatographie par perméation de gel sur une matière échangeuse d'ions.
